# EUROPEAN PATENT SPECIFICATION

(11) **EP 1 933 144 B1**
(45) Date of publication and mention of the grant of the patent: **28.09.2011**
(21) Application number: 06025527.0
(22) Date of filing: 11.12.2006
(51) Int. Cl.: G01N 33/558, G01N 33/543, G01N 33/58, C12Q 1/68

(54) **Rapid immunochromatographic detection by amplification of the colloidal gold signal**
Schnelle immunochromatographische Detektion durch Verstärkung des kolloidalen Gold Signals
Détection rapide immunochromatographique par amplification du signal d'or colloïdal

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- EP-A- 0 590 695
- WO-A-02/46472
- WO-A-2006/039542
- GB-A- 2 284 479
- TANAKA R ET AL: "A novel enhancement assay for immunochromatographic test strips using gold nanoparticles." ANALYTICAL AND BIOANALYTICAL CHEMISTRY, vol. 385, no. 8, August 2006 (2006-08), pages 1414-1420, XP002424110
- OKU Y ET AL: "Development of oligonucleotide lateral-flow immunoassay for multi-parameter detection" JOURNAL OF IMMUNOLOGICAL METHODS, vol. 258, no. 1-2, 1 December 2001 (2001-12-01), pages 73-84, XP004311918
- SIMS P W ET AL: "IMMUNOPOLYMERASE CHAIN REACTION USING REAL-TIME POLYMERASE CHAIN REACTION FOR DETECTION" ANALYTICAL BIOCHEMISTRY, vol. 281, no. 2, 1 June 2000 (2000-06-01), pages 230-232, XP001147707
- HAZARIKA P. ET AL: 'Sensitive detection of proteins using difunctional DNA-gold nanoparticles' SMALL, WILEY - VCH VERLAG GMBH & CO. KGAA, DE vol. 1, no. 8-9, 01 January 2005, pages 844 - 848, XP002407187 ISSN: 1613-6810

## Description

The present invention relates in general to the field of diagnostics, namely to a device for the detection of a target in a sample. More precisely, the present invention relates to a rapid immunochromatographic test device suitable for sensitivity development of an antibody and/or antigen detection. The present invention further refers to a method for the production of the test device, to the uses of the test device for the early detection of disease infection such as HIV in a sample, as well as to a kit comprising the test device.

### BACKGROUND OF THE INVENTION

In recent years the *in vitro* diagnostics (IVD) industry has made enormous efforts to develop immunochromatographic tests. Such tests have found applications in both clinical and non-clinical fields ¹. A clinical utility of this test format has been shown for more than 150 different analytes, and many of them are target now of commercially available diagnostic products ³. The wide range of applications for such devices has been reviewed ^{1,2}.

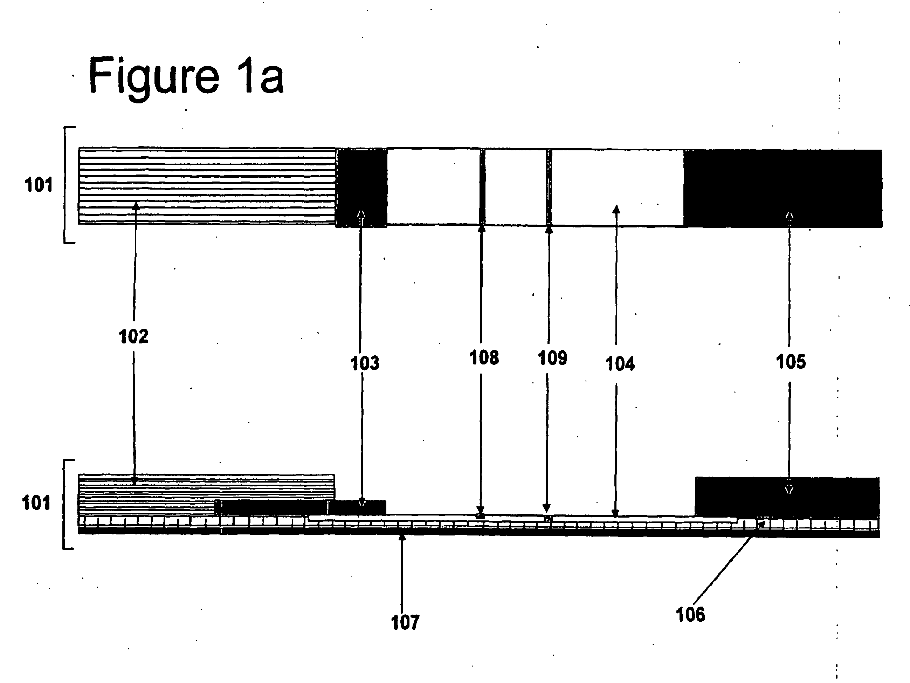

Rapid immunochromatographic test devices, e.g. in the form of a test strip, are made up of a number of components (Figure 1a). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

For example, testing for HIV is an essential component in the diagnosis and treatment of persons infected with the virus, in screening of blood for transfusion, in surveillance and in HIV/AIDS related research. Thus accurate and cost-effective testing is of great importance in combating the spread of HIV. It is imperative that tests for the diagnosis of HIV infection be as accurate as possible, given the serious ethical, legal and social issues that accompany HIV infection.

The number of people living with HIV has now risen to reach its highest level ever: close to 40 million people are living with the virus and close to 5 million people were newly infected with HIV in 2004 alone. Worldwide, the AIDS epidemic killed over 3 million people last year alone (Source: UNAIDS). Furthermore, only one in five people needing HIV prevention worldwide have access to basic prevention services and only one in ten people living with HIV has been tested for the virus.

The HI virus is most easily transmitted to others during the initial period of acute HIV infection, when the viral load (quantity of HIV RNA in the blood) is especially high and when people are not aware of being contaminated by the virus. Most HIV infections are transmitted at this stage, called primary infection. Earlier detection using ultra sensitive tests avoids missing primary infections, enabling immediate precautionary measures to be taken to help prevent the risk of HIV transmission to a non-infected partner, to an unborn child, or through blood donations or direct blood contact. Earlier detection of HIV infection also ensures the implementation of early antiretroviral therapy (ART) to slow down the progression of HIV infection, thereby improving patient care and quality of life.

The diagnosis of HIV infection is usually made on the basis of the detection of HIV antibodies and/or antigen. The diagnosis of an HIV infection can be made indirectly, i.e. through the demonstration of virus-specific antibodies. Besides such indirect diagnosis based on detection of antibodies, a direct diagnosis of HIV infection is also possible: either through the demonstration of infectious virus (using cell culture), viral antigens (p24 antigen ELISA) or viral nucleic acid (i.e. viral genome); the latter is also termed nucleic acid testing (NAT).

One important problem of HIV antibody testing is the so-called "diagnostic window". This is the time period that elapses between the time of acquisition of HIV infection until detectable levels of antibodies are present. The switch from antibody-negative to antibody-positive is called "seroconversion".

The most widely used screening tests are ELISAs as they are the most appropriate for screening large numbers of specimens on a daily basis, e.g. blood donations. The earliest assays used purified HIV lysates (1st generation assays). Improved assays based on recombinant proteins and/or synthetic peptides, which also enabled the production of combined HIV-1/HIV-2 assays, became rapidly available (2nd generation assays). The so-called 3rd generation or antigen-sandwich assays, which use labeled antigens as conjugate, are more sensitive and have reduced the diagnostic window period considerably ^{4,5}.

The window interval between the presence of HIV-1 RNA in plasma and antibody seroconversion varies between 27.4 and 10.2 days depending on the route of infection. HIV infection is detected between 17.4 and 9.4 days earlier by testing for the HIV antigen p24 compared to the above mentioned 3rd generation assays ⁶. Testing for the antigen p24 was the first assay available for the diagnosis of HIV infection prior to antibody seroconversion and has been available commercially since 1986 for the detection of HIV antigen in serum, plasma and cerebrospinal fluid (CSF) using enzyme immunoassay (EIA) technology ⁷⁻¹⁰.

Although the prevalence and incidence of HIV infection in the general population in industrialized countries are relatively low, the residual risk for HIV transmission by blood donation (mostly by viremic but antibody negative donors) is ~ 1/493 000 per unit in the USA ¹¹. Despite the efforts to improve self-exclusion, some donors may not report or not perceive risk behavior for HIV infection ¹². By additional screening for the p24 antigen, the risk of HIV infection may be reduced to 1/676 000. However, the cost-effectiveness of such testing is predicted to be far below that of most medical interventions ¹³. In countries with high transmission rates, i.e. Thailand where the HIV incidence is 10-100 times than that in the USA, p24 antigen testing of donors has been shown to be highly cost-effective ¹⁴.

Furthermore, anti-retroviral therapy is becoming available more readily throughout the developing world as a result of increases in health budgets as well as drastic price reductions negotiated with pharmaceutical companies. Unfortunately, the cost of serological tests required for the follow up of the treatment remains highly prohibitive for the majority of the patients in resource-limited countries. This situation may not only contribute to render anti-retro viral therapy ineffective but also contribute to the emergence of drug-resistant HIV strains ¹⁵. A single PCR-based determination of the RNA viral load can cost US$ 100 or more per test, which is equal to the costs for one month of life-saving treatment for an HIV patient receiving anti-retroviral therapy ¹⁶. The development and implementation of reliable low-cost tools to monitor the effectiveness of treatment is therefore urgently desired.

In recent years, the development of enhanced ELISA assays that detect both HIV antibody and antigen (4th generation assays) has led to earlier detection of HIV seroconversion by further reducing the diagnostic window period. 4th generation HIV assays allow the combined detection of HIV antigen and antibody (Ag/Ab) in order to reduce the diagnostic window between infection and antibody detection in primary HIV infection. These 4th generation HIV screening assays are known in the art since the late 1990s. These assays are reported to reduce the diagnostic window of HIV infection by an average of 7 days in comparison to the 3rd generation HIV screening assays. Therefore, an earlier diagnosis of HIV infection is possible, by detecting p24 antigen which may be present in samples from individuals with recent HIV infection prior to seroconversion.

Several 4th generation HIV screening assays are also commercially available [17]. For example, AxSYM® HIV Ag-Ab (Abbott Laboratories, Abbott Park, USA), Murex HIV Ag/Ab Combination (Abbott/Murex Biotech Ltd., Dartford, UK), Genscreen® Plus HIV Ag-Ab (Bio-Rad Laboratories, Hercules, USA) and Enzymun-Test® HIV Combi (Roche Boehringer Mannheim, Penzberg, Germany). In these commercial assays, the presence of HIV antibodies and p24 antigen is detected at the same time as one combined signal, However, such one combined signal does not allow to discriminate whether the positive signal is based on a detection of antibodies or antigen or both. There further exist for example Vironostika® HIV Uni-Form II Ag/Ab (Organon Teknika, Boxtel, Netherlands) or Enzygnost® HIV Integral (Dade Behring, Mannheim, Germany).

Furthermore, there are the commercially available 4th generation assays of the type VIDAS® HIV DUO, such as VIDAS® HIV DUO and VIDAS® HIV DUO ULTRA (bioMérieux, Lyon, France). They utilize solid-phase receptacles, divided in a lower and an upper part, wherein the lower part detects HIV antibodies and the upper part detects HIV p24, i.e. the presence of HIV antibodies and p24 antigen can be detected as two separate signals. However, the majority of the commercially available 4th generation HIV screening assays still utilize coated microtiter-plates or blends of microparticles (except for assays of the type VIDAS® HIV DUO which utilize solid-phase receptacles).

Recently, a variety of simple, instrument-free initial HIV tests have become available, including agglutination, immunofiltration (flow through tests), immunochromatographic (lateral flow tests) and dipstick tests, which are primarily based on the principle of the 3rd generation assays ¹⁸. Specimen and reagents are often added by means of a dropper to the test device. A positive result is indicated by the appearance of a colored dot or line, or shows an agglutination pattern. Most of these tests can be performed in less than 20 minutes, and are therefore called simple/rapid assays. The results are read visually. Examples for commercially available simple and/or rapid 3rd generation assays are InstantCHEK™-HIV 1+2 (EY Laboratories Inc.), GENIE II HIV-1/HIV-2 (Bio-Rad), Efoora HIV Rapid (Efoora Inc.). OraQuick HIV-1/2 Rapid HIV -1/2 antibody (OraSure Technologies Inc.), SD Bioline HIV 1/2 3.0 (Standard Diagnostics Inc.), Hema·Strip® HIV 1/2 (Chembio Diagnostics), HIV 1/2 STAT-PAK (Chembio Diagnostics), HIV (1+2) Antibody (Colloidal Gold) (KHB Shanghai Kehua Bio-engineering Co. Ltd.), GENEDIA® HIV 1/2 Rapid 3.0 (Green Cross Life Science Corp.), DoubleCheckGold™ HIV 1&2 (Orgenics Ltd.), and Uni-Gold™ HIV 1/2 (Trinity Biotech Ltd., Ireland) [18]. In general, these simple/rapid tests are most suitable in testing and counselling centres and laboratories that have limited facilities and process low numbers of specimens daily.

Canadian patent application CA 2,431,778 discloses a rapid diagnostic immunoassay that belongs to the 4th generation assays since it allows the combined detection of HIV 1+2 antibodies and the HIV antigen p24. This immunoassay comprises a test strip having an upstream and a downstream end, two test zones and one control zone and a housing having opening and/or transparent materials. One of the two test zones of the test strip contains HIV 1+2 antigen for the detection of HIV 1+2 antibodies and the other of the two test zones contains a monoclonal antibody against p24 for the detection of p24 antigen.

However, most commercially available 4th generation HIV screening assays reduce but still cannot close the diagnostic window during primary HIV infection. Therefore, HIV screening assays always need to be judged in the context of symptoms and risk factors. Furthermore, the sensitivity of the p24 antigen detection in 4th generation assays needs to be improved ¹⁹. Therefore, there is a need for further improving 4th generation assays in order to increase the sensitivity as well as the specificity.

Further, there is not only a need to further improve the 4^{th} generation assays, there is the fundamental need to improve the sensitivity as well as the specificity of rapid immunochromatographic test devices which are simple in use. As above in case of HIV outlined the early detection of disease infection is crucial to ensure the implementation of an early therapy and thus to slow down the progression of the infection, thereby improving patient care and quality of life. This applies to the detection of infections such as Hepatitis C, H.Pylori, Hepatitis B, Leishmania, Schistosomiasis, Malaria, Tuberculosis, and in particular HIV.

EP 0 590 695 refers to a liquid transfer device for use in assay procedures comprising a sheet of porous material for capillary liquid flow therethrough.

GB 2 284 479 refers to a liquid transfer device having utility in diagnostic assays comprising first and second capillary flow channels.

WO 2006/039542 refers to an analytical device for performing an assay to determine the presence or approximate quantity of an analyte in a liquid sample, the device comprising primary and secondary flow paths and a capture zone.

Tanaka *et al.* 2006 ²¹ refers to a highly sensitive immunochromatographic assay applied to detect hCG as the model case. Primary antibody-conjugated gold nanoparticles were used as an enhancer. The primary antibodies were immobilized within a defined detection zone (test line) on the diagnostic nitrocellulose membrane. Secondary antibodies were conjugated with colloidal gold nanoparticles. The gold-conjugated antibodies and the primary antibodies formed a sandwich complex with the target protein. Within the test line, the sandwich complex was immobilized.

WO 02/46472 refers to the detection of analytes, in particular nucleic acids. The method comprises contacting the nucleic acid with particles having oligonucleotides attached thereto.

Hazarika *et al.* 2005 ²² refers to the sensitive detection of proteins using an alternative approach based on difunctional DNA-gold nanoparticles.

There is need in the prior art to provide a rapid immunochromatographic test device suitable for the ultra-sensitive detection of target in a sample.

Therefore it is an object of the present invention to overcome the drawbacks of the prior art and to provide especially a simple and rapid test device for the ultra-sensitive antibody and/or antigen detection by signal development and signal amplification suitable to be employed for the early detection of disease infections in a sample.

### SUMMARY OF THE INVENTION

In one embodiment the present invention concerns a rapid immunochromatographic test device for the detection of a target in a sample, comprising
(a) a first gold conjugate releasing pad, comprising a first colloidal gold conjugated with a first antibody or antigen and at least one oligonucleotide, and
(b) a second gold conjugate releasing pad, comprising a second colloidal gold conjugated with a second antibody or antigen and at least one oligonucleotide complementary to the at least one oligonucleotide of the first colloidal gold conjugate;
(c) a sample pad;
(d) a first conjugate pad comprising said first gold conjugate releasing pad;
(e) a second conjugate pad comprising said second gold conjugate releasing pad;
(f) a membrane comprising a capture test zone and a negative control zone; and
(g) an absorbent pad;
wherein said capture test zone comprises said second antibody or antigen;
wherein the second antibody immobilized within the capture test zone captures the target from a site that differs from that site captured by the first antibody;
wherein both releasing pads are located at different positions within the test device; such that the target in the sample will be captured by the first colloidal gold conjugated with the first antibody or antigen to form a complex "target-first colloidal gold conjugate", the complex will be captured then by the second antibody or antigen immobilized within the capture test zone, and then the second colloidal gold conjugated with the second antibody or antigen will be released and
capture the target from a site that differs from that site captured by the first antibody and at the same time with the oligonucleotide(s) that are complementary with the oligonucleotide(s) conjugated with the first colloidal gold.

In another embodiment the present invention relates to a rapid immunochromatographic test device for the detection of a target in a sample, comprising
(a) a first gold conjugate releasing pad, comprising a first colloidal gold conjugated with a first antibody or antigen and at least one further antibody or antigen, wherein said further antibody or antigen differs from said first antibody or antigen, and
(b) a second gold conjugated releasing pad, comprising a second colloidal gold conjugated with a second antibody or antigen and at least one further antibody or antigen, wherein said further antibody or antigen are specific for the at least one further antibody or antigen of the first colloidal gold conjugate;
(c) a sample pad;
(d) a first conjugate pad comprising said first gold conjugate releasing pad;
(e) a second conjugate pad comprising said second gold conjugate releasing pad;
(f) a membrane comprising a capture test zone and a negative control zone; and
(g) an absorbent pad;

In a further embodiment the present invention concerns a method for the production of a device according to the present invention comprising the steps of
(a) preparing a colloidal gold solution;
(b) preparing a conjugation buffer;
(c) partitioning the conjugation buffer by dividing it into a first and a second flask;
(d) adding an antibody according to the present invention to the conjugation buffer in the first flask;
(e) adding an antibody according to the present invention to the conjugation buffer in the second flask, wherein said antibody differs from the antibody used in step d);
(f) preparing and adding oligonucleotides labelled BSA aqueous solution to the first flask;
(g) preparing and adding complementary oligonucleotides labelled BSA aqueous solution to the conjugation buffer in the second flask;
(h) adding colloidal gold solution into each flask;
(i) adding stabilizing buffer to each flask;
wherein said capture test zone comprises said second antibody or antigen;
wherein the second antibody immobilized within the capture test zone captures the I target from a site that differs from that site captured by the first antibody;
wherein both releasing pads are located at different positions within the test device;
such that the target in the sample will be captured by the first colloidal gold conjugated with the first antibody or antigen to form a complex "target-first colloidal gold conjugate", the complex will be captured then by the second antibody or antigen immobilized within the capture test zone, and then the second colloidal gold conjugated with the second antibody or antigen will be released and capture the target from a site that differs from that site captured by the first antibody and at the same time with the further antibody or antigen that are specific for the further antibody or antigen conjugated with the first colloidal gold.
(j) concentrating each conjugate;
(k) adding a surfactant to the first conjugate and soaking glass fibre sheet conjugate pad into the conjugate;
(1) soaking another glass fibre sheet conjugate pad into the second conjugate; j
(m) printing capturing/sample and control lines onto the membrane,
(n) laminating cards using the glass fibre sheet conjugate pad soaked with the first gold conjugate; and
(o) cutting cards into strips.

In a further embodiment the present invention concerns a method for the production of a device according to the present invention comprising the steps of
(a) preparing a colloidal gold solution;
(b) preparing a conjugation buffer;
(c) partitioning the conjugation buffer by dividing it into a first and a second flask;
(d) adding an antibody according to claim 8 to the conjugation buffer in the first flask;
(e) adding an antibody/antigen according to claims 8 and 9 to the conjugation buffer in the second flask, wherein said antibody differs from the antibody used in step d);
(f) preparing and adding aqueous solution comprising antibodies or antigens, wherein said antibodies are different from the antibody used in step d) and e) to the first flask;
(g) preparing and adding aqueous solution comprising antibodies or antigens ; complementary to the antibodies or antigens used in step (f) to the conjugation buffer in the second flask;
(h) adding colloidal gold solution into each flask;
(i) adding stabilizing buffer to each flask;
(j) concentrating each conjugate;
(k) adding a surfactant to the first conjugate and soaking glass fibre sheet conjugate pad into the conjugate;
(l) soaking another glass fibre sheet conjugate pad into the second conjugate;
(m) printing capturing/sample and control lines onto the membrane,
(n) laminating cards using the glass fibre sheet conjugate pad soaked with the first gold conjugate; and
(o) cutting cards into strips.

In another embodiment the present invention relates to the use of a device according to the present invention for the detection of a disease in at least one sample.

In a further embodiment the present invention refers to a kit for detection of a disease comprising the device according to the present invention and a manual.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

As outlined above there is a need in the prior art to provide a new test device suitable for the early detection of a disease infection in a sample. There is also a need in the art for devices suitable for simple, rapid and ultra-sensitive detection of an antigen and/or antibody, which devices having a higher sensitivity than devices from the prior art.

In a first aspect the present invention provides a rapid immunochromatographic test device for the detection of a target in a sample as defined in claim 1.

The first colloidal gold conjugated with a first antibody or antigen captures the target in the sample and forms a complex "target-first colloidal conjugate". Preferably this target in the sample is an antigen and/or antibody.

In a preferred embodiment of the device according to the present invention each gold conjugate comprises between 1 and 6 different oligonucleotides. Preferably each gold conjugate comprises between 2 and 4 different oligonucleotides. These oligonucleotides usually have a length of about 15 to 25 nucleotides, preferably of about 20 nucleotides. Further these oligonucleotides have an amino group at the 5' terminus, preferably conjugated with bovine serum albumin. The bonds formed between the gold and oligonucleotides are the same bonds as those usually formed between gold and antibodies/antigens, and which are known to a person skilled in the art. Preferably such bonds are driven by hydrophobic, hydrophilic and dative binding forces.

In another preferred embodiment of the device according to the present invention the rapid immunochromatographic test device for the detection of a target in a sample, comprises
a) a first gold conjugate releasing pad, comprising a first colloidal gold 201 conjugated with a first antibody 202 or antigen and four different oligonucleotides 204, 205, 206, 207, and
b) a second gold conjugate releasing pad, comprising a second colloidal gold 211 conjugated with a second antibody 203 or antigen and four different oligonucleotide 203', 204', 205', 206' complementary to the four oligonucleotides 204, 205, 206, 207 of the first colloidal gold conjugate 201.

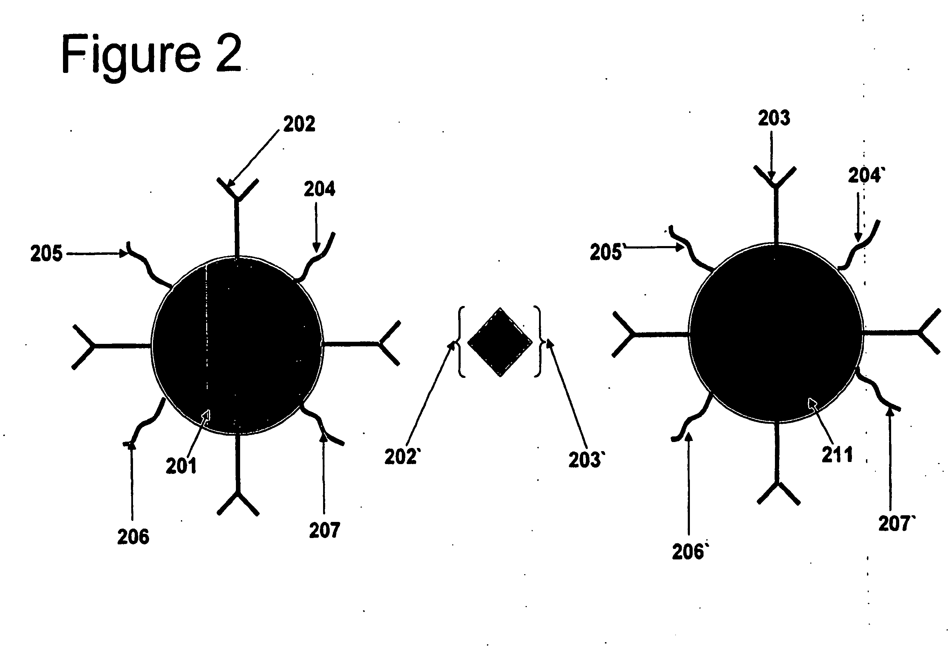

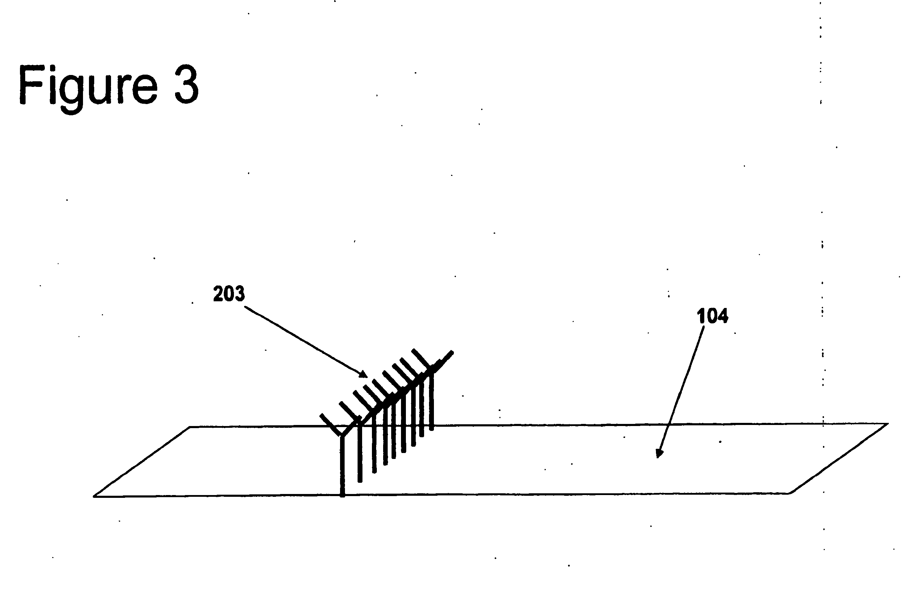

The first gold conjugate releasing pad of the device according to the present invention comprises a gold conjugate 201 that is conjugated with a first specific antibody 202 or antigen to capture the target analyte from the first site 202' and (at the same time) with some specific oligonucleotides. The second gold conjugate releasing pad comprises a gold 211 conjugated with a second specific antibody 203 or antigen to capture the target analyte from the second site 203' and at the same time with the complementary oligonucleotides that are conjugated with the first colloidal gold conjugate (figure 2). The last mentioned conjugated antibody 203 or antigen is the same antibody or antigen that is immobilized onto the nitrocellulose membrane (figure 3).

In a second aspect the present invention provides a rapid immunochromatographic test device for the detection of a target in a sample as defined in claim 2.

In a preferred embodiment of the device according to the present invention specific antibodies or antigens (which should differ than that of first and second specific antibody or antigen) with their specific antigens or antibodies could be employed to play the same role of the oligonucleotides and their complementary oligonucleotides.

The device according to the present invention comprises a test strip comprising
a) a sample pad,
b) a conjugate pad comprising the first gold conjugate releasing pad,
c) a conjugate pad comprising the second gold conjugate releasing pad,
d) a membrane comprising a capture test zone and a negative control zone, and
e) an absorbent pad.

The capture test zone of the device according to the present invention comprises the second antibody or antigen. The antibody immobilized within the test zone captures the target from a site that differs from that site captured by the first antibody conjugated with the first colloidal gold, why both antibodies differ from each other.

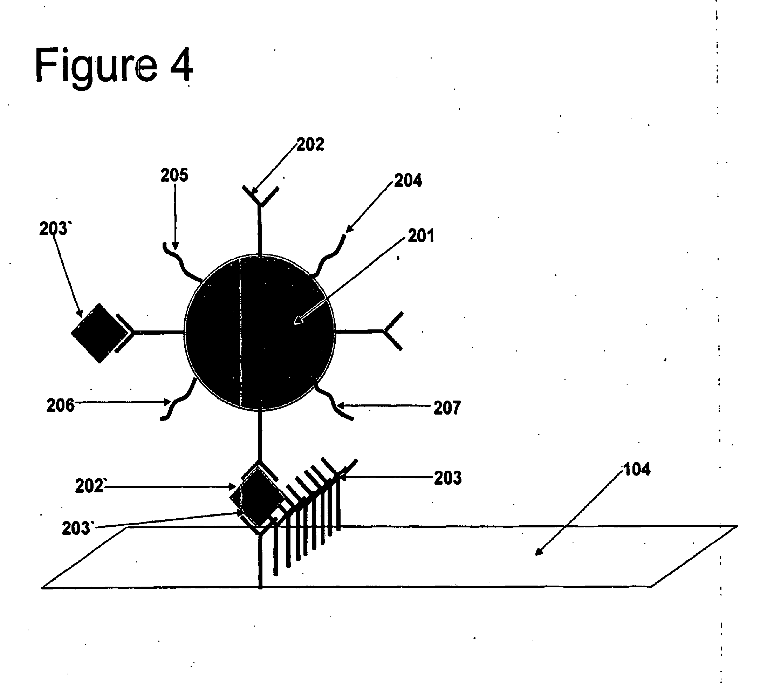

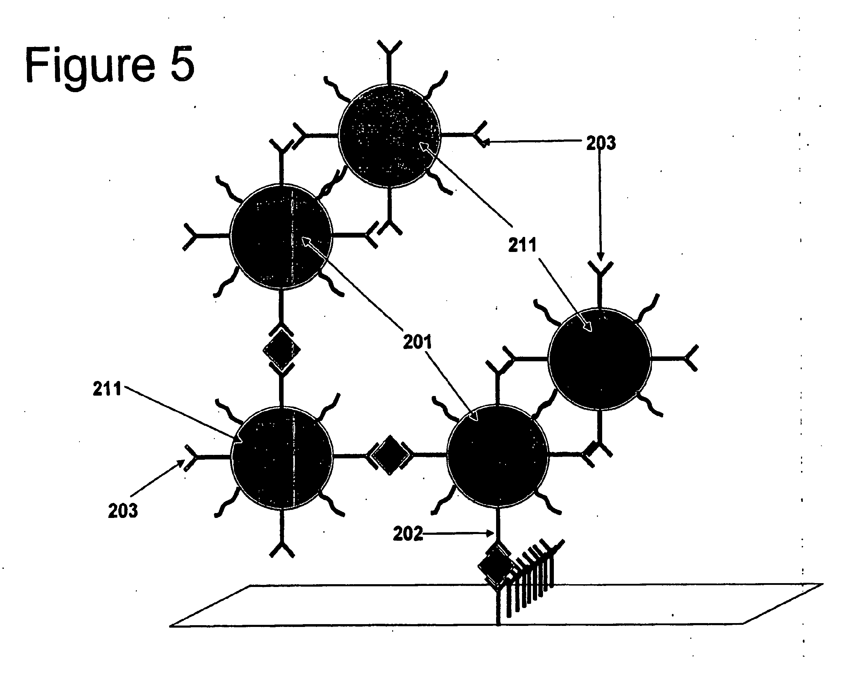

The second specific antibody or antigen is also immobilized within the test zone 108. The complex "target-first colloidal gold conjugate" will be captured by this second antibody 203 or antigen and therefore kept within the test zone 108 to form the sandwich detection (figure 4). Then, the second gold conjugate releasing will release its gold 211 conjugated with the second specific antibody 203 or antigen to capture the target analyte from the second site 203' and at the same time with the complementary oligonucleotides 204', 205', 206', 207' that are complement the oligonucleotides 204, 205, 206, 207 conjugated with the first colloidal gold conjugate 201. The second conjugate 211 would bind with the first conjugate from different sites, this binding could be happened between any of the conjugated oligonucleotides with its complementary oligonucleotide on the other gold conjugate or between any free site 203' of the analyte with its specific antibody or antigen on the second conjugate (figure 5). Nevertheless, the other oligonucleotides will be able to link with their complementary oligonucleotides beside the probability of capturing the first conjugate that will capture the second conjugate to form more and more-branched bonds that propagate the accumulation of colloidal gold particles onto the capturing/sample line (figure 5). This propagation and accumulation of colloidal gold signal will amplify the signal and highly increase the sensitivity. This will enable us to detect very low concentrations that are not detectable using the same technique without signal amplification.

In one embodiment of the device according to the present invention the membrane is attached by means of an adhesive to a supporting backing. Preferably an acrylic pressure sensitive adhesive as known in the art is used.

In another embodiment of the device according to the present invention the first and second gold conjugate pad are laminated between the sample pad and the membrane, wherein the two gold conjugates are separated by a divider.

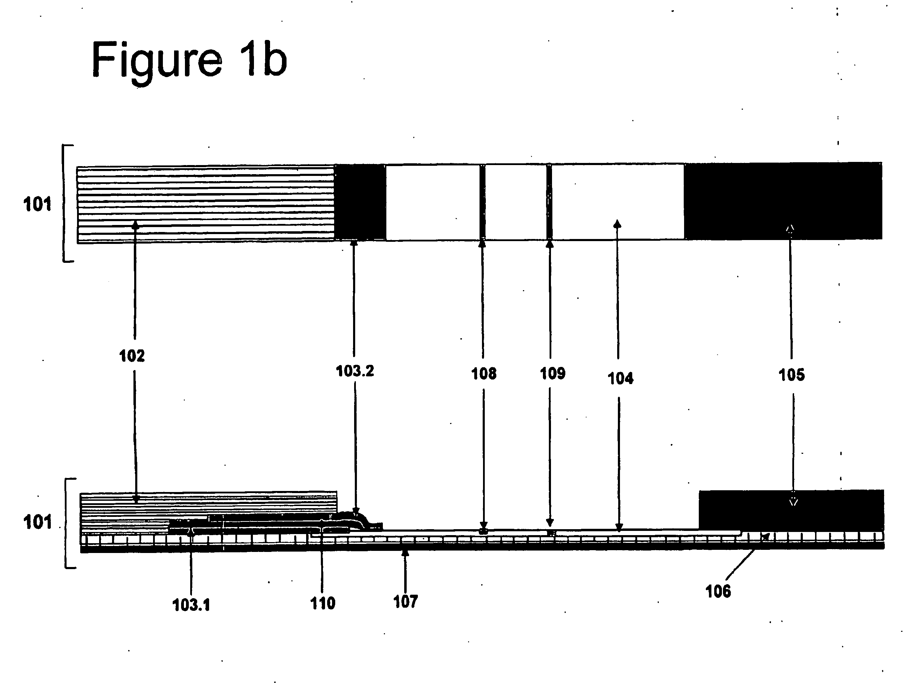

In a preferred embodiment of the device according to the present invention the first 103.1 and second gold conjugate pad 103.2 are laminated between the sample pad 102 and the membrane 104, wherein the two gold conjugates are separated by a divider 110 (Figure 1b). Preferably, the divider is an inert divider, more preferably the divider is a plastic divider

In another embodiment, the device according to the present invention the first gold conjugate pad is attached between the sample pad and the membrane while the second gold conjugate pad is within the upper part of the plastic housing to be released after sample application onto the nitrocellulose membrane directly.

In one preferred embodiment of the device according to the present invention the supporting backing is a plastic backing.

In another preferred embodiment of the device according to the present invention the membrane is nitrocellulose membrane.

In one embodiment of the device according to the present invention the first or second antibody is selected from the group comprising mouse anti-HIV p24, mouse anti-HBsAg, anti-hlgG, anti-Lipoarabinomannan, anti-H.pylori antigen, anti-Leishmania antigen, anti-Pneumonia-antigen, anti-Malaria antigen, anti-Chlamydia antigen, anti-Toxoplasma antigen, anti-Schistosoma antigen , HIV 1 antibody, and HIV 2 antibody.

In a preferred embodiment of the device according to the present invention the first or second antibody is a monoclonal or polyclonal antibody, preferably a monoclonal antibody.

In another embodiment of the device according to the present invention the first antigen is selected from the group comprising conjugate of HIV antigen, conjugate of hepatitis C antigen, HIV 1 antigen, HIV 2 antigen, Lipoarabinomannan, H.pylori antigen, Toxoplasma antigen.

In a further embodiment of the device according to the present invention the control zone comprises a non-specific capturing antibody and/or a non-specific antibody capturing protein.

In one preferred embodiment of the device according to the present invention the non-specific antibody is selected from the group consisting of anti-mouse IgG, anti-rabbit IgG, anti-goat IgG, anti-donkey IgG, Anti-sheep IgG, anti-HIV p24, anti-Lipoarabinomannan, anti-H.pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

In another preferred embodiment of the device according to the present invention the non-specific capturing protein is either Protein A or Protein G.

In a preferred embodiment of the device according to the present invention the device comprises a housing comprising at least one test strip according to the present invention.

In another preferred embodiment of the device according to the present invention the housing comprises two, three, four, five, six, seven, eight, nine, or ten test strips. Preferably the housing comprises two, three, four, or five test strips, more preferably the housing comprises two or three test strips.

In one preferred embodiment of the device according to the present invention each test strip contains at least two antibodies or antigens, or at least one antibody and one antigen, wherein one of these antibodies or-antigens is immobilized onto the membrane and the other one is conjugated with the first colloidal gold. In case of two antibodies, they have to be different to capture the target from two different sites.

In another aspect the present invention concerns a method for the production of a device according to the present invention, comprising the steps of
a) preparing a colloidal gold solution;
b) preparing a conjugation buffer;
c) partitioning the conjugation buffer by dividing it into a first and a second flask;
d) adding an antibody according to the present invention to the conjugation buffer in the first flask;
e) adding an antibody according to the present invention to the conjugation buffer in the second flask, wherein said antibody differs from the antibody used in step d);
f) adding colloidal gold solution into each flask;
g) adding stabilizing buffer to each flask;
h) concentrating each conjugate;
i) adding a surfactant to the first conjugate and soaking glass fibre sheet conjugate pad into the conjugate;
j) soaking another glass fibre sheet conjugate pad into the second conjugate;
k) printing sample and control lines onto the membrane;
L) laminating cards using the first gold conjugate; and
m) cutting cards into strips.

The method according to the present invention further comprises either the steps of
a. preparing and adding oligonucleotides labelled BSA aqueous solution to the first flask;
b. preparing and adding complementary oligonucleotides labelled BSA aqueous solution to the conjugation buffer in the second flask;
wherein the solutions are added before step f) according to the method of the present invention.

or the method according to the present invention further comprises the steps of
a) preparing and adding aqueous solution comprising antibodies or antigens, wherein said antibodies are different from the antibody used in step d) and e) according to the method of the present invention to the first flask;
b) preparing and adding aqueous solution comprising antibodies or antigens complementary to the antibodies or antigens used in step a) to the conjugation buffer in the second flask;
wherein the solutions are added before step f) according to the method of the present invention.

In another aspect the present invention relates to the use of a device according to the present invention for the detection of a disease in at least one sample.

In one preferred embodiment of the use according to the present invention the antibody in one sample (e.g. specimen) and the antigen in another sample (e.g. specimen) is detected. For example, in the case two test strips are used, Lipoarabinomannan-antigen can be detected in urine, while anti-lipoarabinomannan is detected in serum (figures 4 a and b).

In another preferred embodiment of the use according to the present invention the antibody and antigen are detected in the same sample (specimen). For example, HIV antibodies and the HIV p24 antigen are detected in the same serum sample (specimen) using a device of two different strips (figures 5 a and b).

In one embodiment of the use of the device according to the present invention the sample was obtained from a human.

In one preferred embodiment of the use of the device according to the present invention the sample is selected from the group comprising of whole blood, serum, plasma, saliva, and urine.

In another preferred embodiment of the use of the device according to the present invention the disease detected in said sample is selected from the group consisting of HIV, Hepatitis A, Hepatitis B, Hepatitis C, H.pylori, Leishmania, Schistosomiasis, Malaria, Pneumonia, Toxoplasmosis, Tubercolosis and Chlamydial infection.

In a further aspect the present invention refers to a kit for detection of a disease comprising the device according to the present invention and a manual.

In one preferred embodiment of the kit according to the present invention the kit further comprises an assay buffer. The assay buffer can be any buffer known in the art suitable for the use of whole blood samples. Preferably in the case of whole blood samples Tris buffer is used, more preferably 0.1M Tris buffer having a pH of 7.5 and comprising a preservative. Any preservative known by a person skilled in the art can be used, preferably sodium azide and even more preferably 0.01M sodium azide is used.

The following example illustrate the present invention without, however, limiting the same thereto.

### BRIEF DESCRIPTION OF THE DRAWING

**Figure 1a****:** shows top and side views of a typical rapid-flow immunochromatographic test device known in the art in the form of a test strip 101 comprising a sample pad 102, a conjugate pad 103, a membrane 104, an absorbent pad 105, an adhesive 106, a supporting backing 107, a test zone 108, and a control zone 109.
**Figure 1b****:** shows top and side views of a preferred embodiment of a rapid-flow immunochromatographic test device according to the present invention in the form of a test strip 101 comprising a sample pad 102, a first conjugate pad 103.1, a second conjugate pad 103.2, a membrane 104, an absorbent pad 105, an adhesive 106, a supporting backing 107, a test zone 108, a control zone 109, and the conjugates divider 110.
**Figure 2****:** shows the schematically view of a preferred embodiment of the first and second colloidal gold according to the present invention, wherein the first colloidal gold 201 is conjugated with a first antibody 202 and four different oligonucleotides 204, 205, 206, 207 and wherein the second colloidal gold 211 is conjugated with a second antibody 203 and four oligonucleotides 204', 205', 206', 207' complementary to the oligonucleotides of the first colloidal gold 201. In addition, the target is shown having two sides 202' and 203'. The first side 202' of the target is captured by the first antibody 202 of the first gold conjugate 201 and the second side 203' of the target is captured by the second antibody 203 conjugated with the second gold conjugate 211.
**Figure 3****:** shows a simplified scheme of a preferred embodiment of the test device according to the present invention. It shows the test zone 108 of the nitrocellulose membrane 104 on the test strip(101. Wherein the second specific antibody or antigen 203 is immobilized to the test zone 108.
**Figure 4****:** shows the main principle of a preferred embodiment of the signal development according to the present invention. By the sample flow within the rapid immunochromatographic test device the target in the sample will be captured by the first specific antibody or antigen 202 of the first colloidal gold 201 to form the complex "target-first colloidal gold". This complex flows to the test zone 108, where it will be captured by the second specific antibody 203 or antigen that is immobilized onto the nitrocellulose membrane 104 to form a sandwich detection.
**Figure 5****:** shows the main principle of a preferred embodiment of the signal amplification and multiplication according to the present invention. By the sample flow within the rapid immunochromatographic test device the target in the sample will be captured by the first specific antibody 202 or antigen that is conjugated to the first colloidal gold 201 to form the complex "target- first colloidal gold". This colloidal gold 201 is further conjugated to four different oligonucleotides 204, 205, 206, and 207. This complex flows to the test zone 108), where it will be captured by the second specific antibody 203 or antigen that is immobilized onto the nitrocellulose membrane 104. Then, the second colloidal gold 211 conjugated with the second specific antibody 203 or antigen and with the complementary oligonucleotides 204', 205', 206', 207' will be released and bind to the first conjugate 201 from the oligonucleotide(s) side as well as to the colloidal gold conjugate antibody/antigen side(s). These multi binding sites between the two conjugates will propagate and accumulate the colloidal gold particles that enhance the signal.

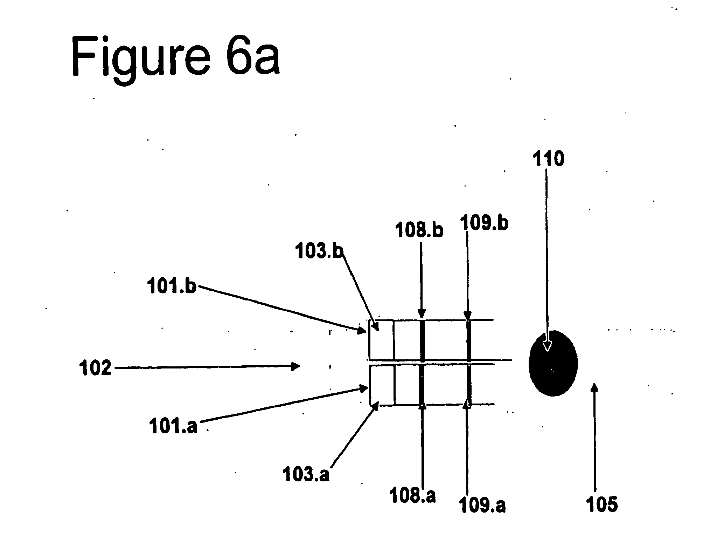

**Figure 6a****:** shows an internal view of a preferred embodiment of a test device according to the present invention suitable to detect two different targets within the same sample (specimen) from the same person (patient). In this preferred embodiment the device comprises two test strips 101.a and 101.b, wherein both test strips share the same sample pad 102, the same humidity indicator 110 and the same absorbent pad 105. Further each test strip 101.a and 101.b comprises its own gold conjugate 103.a and 103.b, sample line 108.a and 103.b and control line 109.a and 109.b.

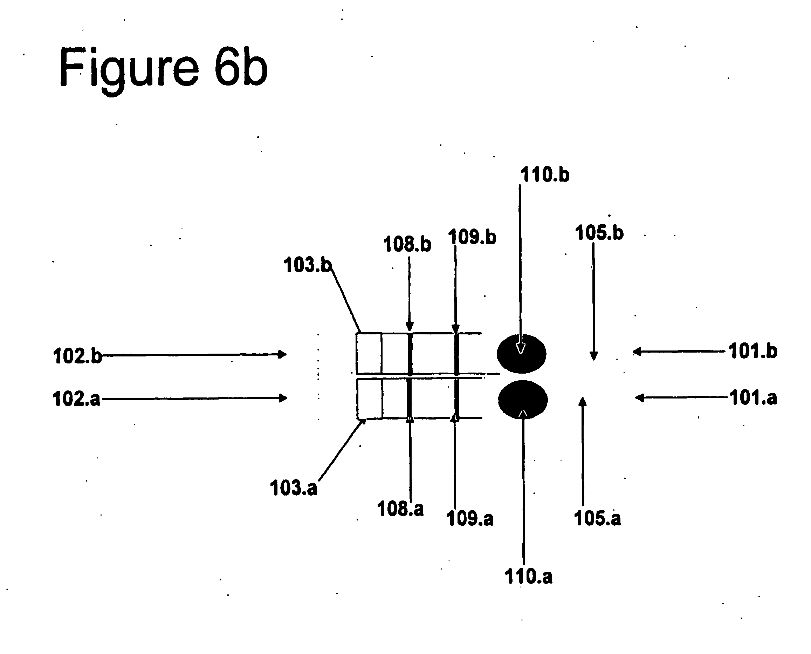

**Figure 6b****:** shows an internal view of a preferred embodiment of a test device according to the present invention suitable to detect two different targets within two different samples (specimens) from the same person (patient). In this preferred embodiment the device comprises two test strips 101.a and 101.b, wherein each of these two strips comprises its own sample pad 102.a and 102.b, gold conjugate 103.a and 103.b, sample line 108.a and 103.b, control line 109.a and 109.b, humidity indicator 110.a and 110.b, and absorbent pad 105.a and 105.b.

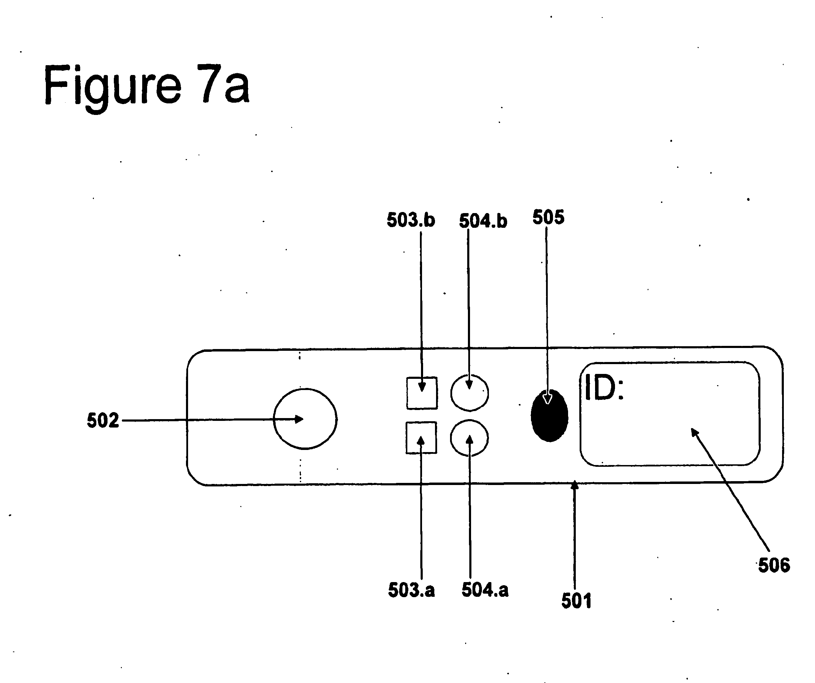

**Figure 7a**: shows an external view of a preferred embodiment of a test device 501 according to the present invention suitable to detect two targets within the same sample (specimen) from the same person (patient). In this preferred embodiment the device 501 comprises a sample application window 502, two test result windows 503.a and 503.b, two control result windows 504.a and 504.b, humidity indication window 505 and a patient ID area 506.

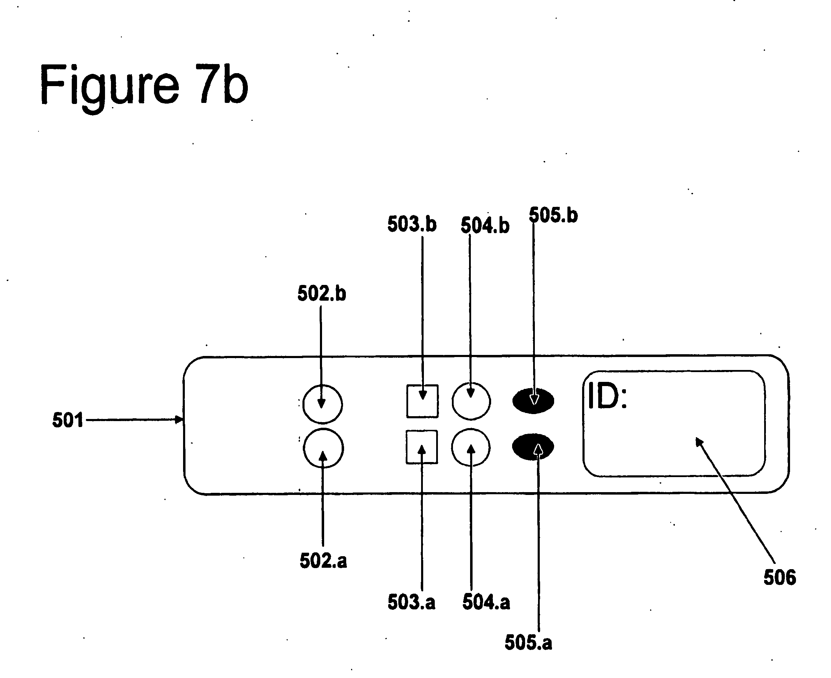

**Figure 7b**: shows an external view of a preferred embodiment of a test device 501 according to the present invention suitable to detect two targets within two different samples (specimens) from the same person (patient). In this preferred embodiment the device 501 comprises two sample application windows 502.a and 502.b, two test result windows 503.a and 503.b, two control result windows 504.a and 504.b, two humidity indication windows 505.a and 505.b and a patient ID area 506.

### EXAMPLES

### Example 1: Preparation of oligonucleotide- and complementary oligonucleotide labeled bovine serum albumin:

5mg of bovine serum albumin (BSA) was linked to each oligonucleotide and another 5mg to the complementary oligonucleotide. Every oligonucleotide had a length of about 20 nucleotides having an amino group at the 5' terminus. The procedure was performed according to the method described by Duncan et al. 1983 ²⁰ comprising the following steps:

### Example 2: Preparation of an preferred embodiment of a test device according to the present invention

The oligonucleotide and complementary oligonucleotide linked BSA prepared as described in Example 1 are further processed according to a procedure comprising the following steps:
a) prepare oligonucleotide linked BSA solution, according to example 1 (solution 1);
b) prepare complementary oligonucleotide linked BSA solution, according to example 1 (solution 2); 
PROTEIN-NH₂
NH₂-OLIGONUCLEOTIDE
+
+
O
O
O
O
N-O-CO-CH₂-S-CO-CH₃
N-(CH₂)₅-CO-O-N
O
O
O
O
N-succinimidyl
S-acetylthioacatate
N-(6-maleimidoca-
proyloxy) succinimide
PROTEIN-NH-CO-CH₂-S-CO-CH₃
NH₂OH
O
N-(CH₂)₅-CO-NH-OLIGONUCLEOTIDE
O
PROTEIN-NH-CO-CH₂-SH
O
PROTEIN-NH-CO-CH₂-S-
N-(CH₂)₅-CO-NH-OLIGONUCCLEOTIDE
O
Preparation of oligonucleotide-labeled protein
c) prepare 1% aqueous solution of tetrachloroauric acid at room temperature;
d) prepare 4% trisodium citrate aqueous solution at room temperature;
e) prepare 0.05 M Potassium Carbonate aqueous solution at room temperature;
f) prepare 600ml of phosphate stabilizing buffer of pH 7.4, containing BSA, Tween 20, Sucrose, polyvinylpurrolidone and a preservative, e.g. sodium azide, at room temperature;
g) prepare colloidal gold solution by reduction of 1.7 ml boiling tetrachloroauric acid solution (after dilution into 100ml) using 1ml trisodium citrate solution and let it takes the room temperature;
h) dilute the colloidal gold solution as 1:1 using distilled water. Adjust the pH to 7.4 using potassium carbonate solution at room temperature;
i) prepare 200ml of phosphate conjugation buffer of pH 7.4 at room temperature;
j) partition the 200ml conjugation buffer by dividing it into two flasks (100ml of each);
k) add 0.5 mg of aqueous antibody (e.g. anti-hIgG or anti-p24 1^{st} clone) to the conjugation buffer in the first flask with stirring at room temperature;
l) add 0.5 mg of oligonucleotides labelled BSA aqueous solution (solution 1) to the first flask at room temperature;
m) add 0.5 mg of aqueous antibody (e.g. HIV p160 or anti-p24 2^{nd} clone) to the conjugation buffer in the second flask with stirring at room temperature;
n) add 0.5 mg of complementary oligonucleotides labelled BSA aqueous solution (solution 2) to the conjugation buffer in the second flask with stirring at room temperature;
o) add 100ml colloidal gold solution into each flask with stirring at room temperature;
p) after about 45 minutes; add 200ml of stabilizing buffer to each flask;
q) after about 20 minutes; concentrate each conjugate by cooled (temperature around 15°C ) high speed centrifugation (10,000 rpm for one hour);
r) discard the supernatant and re-suspend the concentrated conjugates using the stabilising buffer at room temperature;
s) adjust the concentration for each of the two conjugates to O.D.₅₂₀=2.0;
t) add 0.1ml of Tween 20 to the first conjugate and soak glass fibre sheet conjugate pad into the conjugate, then heat dry at temperature around 50°C; and
u) soak another glass fiber sheet conjugate pad into the second conjugate, then heat dry at temperature around 50°C.
(* In case of antibodies/antigens and their specific antigens/antibodies there is no need for these steps of bovine serum albumin or any other protein labelling. ** Other proteins or peptides could be used other than bovine serum albumin).

Additionally, print sample (e.g. anti-HIV p24, 2^{nd} clone) and control lines (e.g. anti-mouse IgG) onto nitrocellulose membrane, then heat dry at temperature around 50°C.

Finally, laminate cards according to the following procedure:

### A. In case of conjugate releasing site laminated within the upper side of the device plastic housing

Lamination of cards using the first gold conjugate. Laminate card components onto the backing material with the sequence:
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane; and
4. laminate the sample pad.

### B. In case of conjugate releasing site laminated onto the test strip itself separated from the first conjugate by a divider

Laminate card components onto the backing material with the sequence (see figure 1b):
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane;
4. laminate the plastic divider onto the first conjugate (overlaps from the nitrocellulose membrane side);
5. laminate the second conjugate pad onto the divider (overlaps from the nitrocellulose membrane side);
6. laminate the sample pad onto the other end of the card, the sample pad will overlaps with the two conjugate pads and; and
7. then cut cards into strips.

### C. Alternatively

Lamination of the second gold conjugate could be applied within the plastic housing itself to ensure that the two conjugates will not propagate before release from the releasing pad and so stick within the releasing pad.

### Example 3: HIV p24 antigen detection system

The first gold conjugate 103.1 is a conjugate of mouse anti-HIV p24, 1^{st} clone (please note that the numbering of clones are only for explanation and to recognize that always two different clones of monoclonal antibodies were used; these two monoclonal antibodies capture the target antigen from two different sites, why they were called as a pair of monoclonal antibodies by the inventors) and four oligonucleotides, and the second gold conjugate is the conjugate of the mouse anti-HIV p24, 2^{nd} clone and the four complementary oligonucleotides. The first conjugate releasing pad 103.1 is laminated on the test strip between the sample pad 102 and the nitrocellulose membrane 104 while the second 103.2 is above the first conjugate pad 103.1 separated by a divider 110 to be released directly toward the nitrocellulose membrane 104 without flow through the first conjugate pad 103.1 to avoid interact with the first conjugate before reaching the membrane 104 (see figure 1b). The second conjugate releasing site 103.2 could be laminated within the upper side of the device plastic housing, whereas the plastic housing is the plastic design where the test strip is finally inserted. The sample line, more precisely the capture test zone 108, is a mouse anti-HIV p24 2^{nd} clone immobilized onto the nitrocellulose membrane 104. The control line 109 is anti-mouse IgG. Sample 108 and control 109 lines turn into purple color in case of HIV p24 antigen availability in the sample; only the control line 109 turns into purple color in case of HIV p24 antigen free sample (Figure 1b).

There aren't any commercially available rapid tests sensitive enough for HIV p24 antigen detection in early stages of infection with concentrations less than 10pg/ml while according to this system it is so simple to detect it.

### Example 4: Hepatitis B surface antigen (HBsAg) detection system

The first gold conjugate is mouse anti-HBsAg (clone 1) and four oligonucleotides conjugated with colloidal gold conjugate, and the second gold conjugate is the conjugate of the four complementary oligonucleotides. The first conjugate releasing pad 103.1 is laminated on the test strip between the sample pad 102 and the nitrocellulose membrane 104 while the second 103.2 is above the first conjugate pad 103.1 separated by a divider 110 to be released directly toward the nitrocellulose membrane 104 without flow through the first conjugate pad 103.1 to avoid interact with the first conjugate before reaching the membrane, (figure 1b). The second conjugate releasing site 103.2 also could be laminated within the upper side of the device plastic housing.

The sample line 108 is mouse anti-HBsAg (clone2) immobilized onto the nitrocellulose membrane 104. The control line 109 is anti-mouse IgG. Sample 108 and control lines 109 turn into purple color in case of HBsAg availability in the sample; only the control line 109 turns into purple color in case of HBsAg free sample, see Figure1b.

The commercially available rapid tests sensitivity for Hepatitis B surface antigen is within the range 500-1000pg/ml while according to this system it is so simple to detect less than 10 pg/ml.

### Example 5: Human Immunodeficiency Virus (HIV) antibodies detection system

The first gold conjugate 201 is the conjugate of mouse anti-human Immunoglobulin G (anti-hIgG) and four oligonucleotides, and the second gold conjugate 211 is the conjugate of the HIV antigen and the four complementary oligonucleotides. The first gold conjugate pad 103.1 is laminated in the side of nitrocellulose membrane 104 while the second gold conjugate pade 103.2 is laminated above the first pad 103.1 separated by a divider 110 that enables the second conjugate to take a part of the sample and release directly onto the nitrocellulose membrane 104.

The sample line 108 is a combination of synthetic/recombinant HIV antigen immobilized onto the nitrocellulose membrane 104. The control line 109 is anti-mouse IgG. Sample 108 and control 109 lines turn into purple color in case of HIV antibodies availability in the sample; only the control line 109 turns into purple color in case of HIV antibodies free sample,(Figure 1b).

According to this system it is so simple to detect very low titers of HIV antibodies in serum.

### Example 6: Human Immunodeficiency Virus (HIV) antibody / antigen detection system (the 4^{th} generation)

The system is a test device comprises a housing which comprises two test strips 101 as a combination of Examples 3 and 5, wherein each test strip 101 comprises at least one sample application site 102, at least one test zone 108 and one control zone 109.

According to this system it is so simple to detect very low titers of HIV antibodies parallel with the detection of very low concentrations of HIV p24 antigen and so early detection of HIV infection.

### Example 7: Human Immunodeficiency Virus (HIV) antibody detection in saliva

The system is the same as that in Example 5 with some modifications on the system due to the difference of samples composition and properties. Instead of the cellulose material (as used in urine detection strip) fiber glass material is used as a sample pad to increase the ability of movement of the viscose saliva sample. Due to the higher concentration of IgG in saliva compared to that of urine the concentration of the capture antibody/antigen on the nitrocellulose membrane for urinary detections is increased. In addition, the concentration of sample pad buffer used for the urinary detection is different from used for oral detection, i.e. it is about double of concentration in urinary detection systems.

According to this system it is so simple to detect very low titers of HIV antibodies in saliva.

### Example 8: Human Immunodeficiency Virus (HIV) antibody detection in urine

The system is the same as that in Example 5 and 7 with some modifications on the system due to the difference of samples composition and properties. According to this system it is so simple to detect very low titers of HIV antibodies in urine.

### Example 9: Hepatitis C Virus (HCV) antibody detection in saliva

The system is the same as that in Example 7 except the sample line that contains hepatitis C antigens instead of HIV antigens. According to this system it is so simple to detect very low titers of HCV antibodies in saliva.

### Example 10: Hepatitis C Virus (HCV) antibody detection in urine

The system is the same as that in Example 9 except the sample line that contains hepatitis C antigens instead of HIV antigens. According to this system it is so simple to detect very low titers of HCV antibodies in urine.

### Example 11: H.Pylori antibody detection in urine

The system is the same as that in Examples 9 and 10 except the sample line that contains H.Pylori antigens instead of HIV or HCV antigens. According to this system it is so simple to detect very low titers of HCV antibodies in urine.

### References

(1) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3) TC Tisone et al., "Image Analysis for Rapid-Flow Diagnostics", IVD Technology 5, no. 5 (1999): 52-58; http://www.devicelink.com/ivdt/archive/99/09/010.html.
(4) Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
(5) Constantine, N.T., van der Groen, G., Belsey, E.M., Tamashiro, H. (1994) Sensitivity of HIV-antibody assays determined by seroconversion panels. AIDS 8, 1715-1720.
(6) Satten, G.A., Busch, M.P., et al. (1997) Effect of transmission route on window period estimates. Fourth Conference on Retroviruses and Opportunistic Infections, Washington DC, Abstract 122.
(7) WHO. Rapid HIV tests: Guidelines for use in HIV testing and counseling services in resource-constrained settings. Geneva 2004. http://www.who.int/hiv/pub/vct/rapidhivtests/en/
(8) Holodniy M, et al. (1991) Reduction in plasma human immunodeficiency virus ribonucleic acid following dideoxynucleoside therapy as determined by the polymerase chain reaction. J Clin. Invest 88, 1755-1759.
(9) Katzenstein D.A., et al. (1994) Quantitation of human immunodeficiency virus by culture and polymerase chain reaction in response to didanosine after long-term therapy with zidovudine. J. Infect. Dis. 169, 416-419.
(10) Jackson JB, et al. (1998) Practical diagnostic testing for human immunodeficiency virus. Clin. Microb. Rev. 1, 124-138.
(11) Goudsmit J, et al. (1986) Expression of human Immunodeficiency virus antigen (HIV-Ag) in serum and cerebrospinal fluid during acute and chronic infection. Lancet 2, 177-180.
(12) Aubuchon, J.P., Birkmeyer, J.D., Busch, M.P. (1997) Cost-effectiveness of expanded human immunodeficiency virus-testing protocols for donated blood. Transfusion 45, 45-51.
(13) Ward, J.M., Holmberg, S.D., Allen, J.R., Cohn, D.L., et al. (1988) Transmission of human immunodeficiency virus (HIV) by blood transfusion screened as negative for HIV antibody. *N. Engl. J. Med.* **8**, 473-478.
(14) Alter, H.J., et al. (1990) Prevalence of human immunodeficiency virus type 1 p24 antigen in U.S. blood donors - an assessment of the efficacy of testing in donor screening. *N. Engl. J. Med* **323**, 1312-1317.
(15) Mayers, D.L. (1998) Drug-resistant HIV-1. The virus strikes back. *JAMA* **279**, 2000-2002.
(16) Stephenson, J. (2002) Cheaper HIV drugs for poor nations bring a new challenge: monitoring treatment. *JAMA* **288**, 2.
(17) WHO. HIV assays: Operational characteristics *(Phase 1)*. Report 15/ antigen/antibody ELISAs. Geneva 2004.
   http://www.who.int/diagnostics_laboratory/evaluations/hiv/en/
(18) WHO. HIV assays: Operational characteristics *(Phase 1).* Report 14/ simple/rapid tests. Geneva 2003.
   http://www.who.int/diagnostics_laboratory/evaluations/hiv/en/
(19) Meier, T, et al. (2001) Evidence for a diagnostic window in fourth generation assays for HIV. *J. Clin. Virol.* **23**, 113-116.
(20) Duncan, R.J.S., Weston, P.D., Wrigglesworth, R., 1983. A new reagent which may be used to introduce sulfhydryl groups into proteins, and its use in the preparation of conjugates for immunoassay. Anal. Biochem. 132, 68.
(21) R Tanaka, T Yuhi, N Nagatani, T Endo, K Kerman, Y Takamura, E Tamiya (2006) A novel enhancement assay for immunochromatographic test strips using gold nanoparticles. Anal Bioanal Chem **385**: 1414-1420.
(22) Hazarika, P., Ceyhan, B., Niemeyer, C. M. (2005) Sensitive detection of proteins using difunctional DNA-gold nanoparticles. *Small* **1**, 844-848.

## Claims

1. A rapid immunochromatographic test device for the detection of a target in a sample, comprising
(a) a first gold conjugate releasing pad, comprising a first colloidal gold conjugated with a first antibody or antigen and at least one oligonucleotide, and
(b) a second gold conjugate releasing pad, comprising a second colloidal gold conjugated with a second antibody or antigen and at least one oligonucleotide complementary to the at least one oligonucleotide of the first colloidal gold conjugate;
(c) a sample pad;
(d) a first conjugate pad comprising said first gold conjugate releasing pad;
(e) a second conjugate pad comprising said second gold conjugate releasing pad;
(f) a membrane comprising a capture test zone and a negative control zone; and
(g) an absorbent pad;
wherein said capture test zone comprises said second antibody or antigen;
wherein the second antibody immobilized within the capture test zone captures the target from a site that differs from that site captured by the first antibody;
wherein both releasing pads are located at different positions within the test device; such that the target in the sample will be captured by the first colloidal gold conjugated with the first antibody or antigen to form a complex "target-first colloidal gold conjugate", the complex will be captured then by the second antibody or antigen immobilized within the capture test zone, and then the second colloidal gold conjugated with the second antibody or antigen will be released and capture the target from a site that differs from that site captured by the first antibody and at the same time with the oligonucleotide(s) that are complementary with the oligonucleotide(s) conjugated with the first colloidal gold.

2. A rapid immunochromatographic test device for the detection of a target in a sample, comprising
(a) a first gold conjugate releasing pad, comprising a first colloidal gold conjugated with a first antibody or antigen and at least one further antibody or antigen, wherein said further antibody or antigen differs from said first antibody or antigen, and
(b) a second gold conjugate releasing pad, comprising a second colloidal gold conjugated with a second antibody or antigen and at least one further antibody or antigen, wherein said further antibody or antigen are specific for the at least one further antibody or antigen of the first colloidal gold conjugate;
(c) a sample pad;
(d) a first conjugate pad comprising said first gold conjugate releasing pad;
(e) a second conjugate pad comprising said second gold conjugate releasing pad;
(f) a membrane comprising a capture test zone and a negative control zone; and
(g) an absorbent pad;
wherein said capture test zone comprises said second antibody or antigen;
wherein the second antibody immobilized within the capture test zone captures the target from a site that differs from that site captured by the first antibody;
wherein both releasing pads are located at different positions within the test device; such that the target in the sample will be captured by the first colloidal gold conjugated with the first antibody or antigen to form a complex "target-first colloidal gold conjugate", the complex will be captured then by the second antibody or antigen immobilized within the capture test zone, and then the second colloidal gold conjugated with the second antibody or antigen will be released and capture the target from a site that differs from that site captured by the first antibody and at the same time with the further antibody or antigen that are specific for the further antibody or antigen conjugated with the first colloidal gold

3. The device according to claim 1 or 2, wherein said membrane is attached by means of an adhesive to a supporting backing.

4. The device according to any of claims 1 to 3, wherein said first and second gold conjugate releasing pads are laminated between the sample pad and the membrane, wherein said two gold conjugates are separated by a divider.

5. The device according to any of claims 1 to 3, wherein said first gold conjugate releasing pad is attached between the sample pad and the membrane while the second gold conjugate releasing pad is within the upper part of the plastic housing.

6. The device according to any of claims 3 to 5, wherein said supporting backing is a plastic backing.

7. The device according to any of claims 1 to 6, wherein said membrane is nitrocellulose membrane.

8. The device according to any of claims 1 to 7, wherein said first or second antibody is selected from the group comprising mouse anti-HIV p24, mouse anti-HBsAg, anti-hlgG, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

9. The device according to any of claims 1 to 7, wherein said first antigen is selected from the group comprising conjugate of HIV antigen, conjugate of Hepatitis C antigen, HIV 1 antigen (HIV p160), HIV 2 antigen (HN p36), Hepatitis B antigen, Lipoarabinomannan, H.Pylori antigen, Toxoplasma antigen.

10. The device according to any of claims 1 to 9, wherein said control zone comprises a non-specific capturing antibody and/or a non-specific antibody capturing protein.

11. The device according to claim 10, wherein said non-specific antibody is selected from the group consisting of anti-mouse IgG, anti-rabbit IgG, anti-goat IgG, anti-donkey IgG, Anti-sheep IgG, anti-HIV p24, anti-Lipoarabinomannan, anti-H. pylori antigen, anti-Leishmania antigen, anti-Pneumonia antigen, anti-Malaria antigen, anti- l-Chlamydia antigen, anti-Toxoplasma antigen, anti-Schistosoma antigen, HIV I antibody, and HIV 2 antibody.

12. The device according to claim 10, wherein said non-specific capturing protein selected is either Protein A or Protein G.

13. A method for the production of a device according to any of claims 1, 3 to 12, comprising the steps
(a) preparing a colloidal gold solution;
(b) preparing a conjugation buffer;
(c) partitioning the conjugation buffer by dividing it into a first and a second flask;
(d) adding an antibody according to claim 8 9 to the conjugation buffer in the first flask;
(e) adding an antibody/antigen according to claims 8 and 9 to the conjugation buffer in the second flask, wherein said antibody differs from the antibody used in step d);
(f) preparing and adding oligonucleotides labelled BSA aqueous solution to the first flask;
(g) preparing and adding complementary oligonucleotides labelled BSA aqueous solution to the conjugation buffer in the second flask;
(h) adding colloidal gold solution into each flask;
(i) adding stabilizing buffer to each flask;
(j) concentrating each conjugate;
(k) adding a surfactant to the first conjugate and soaking glass fibre sheet conjugate pad into the conjugate;
(l) soaking another glass fibre sheet conjugate pad into the second conjugate;
(m) printing capturing/sample and control lines onto the membrane,
(n) laminating cards using the glass fibre sheet conjugate pad soaked with the first sold conjugate: and
(o) cutting cards into strips.

14. A method for the production of a device according to any of claims 2 to 12, comprising the steps of
(a) preparing a colloidal gold solution;
(b) preparing a conjugation buffer;
(c) partitioning the conjugation buffer by dividing it into a first and a second flask;
(d) adding an antibody according to claim 8 to the conjugation buffer in the first flask;
(e) adding an antibody/antigen according to claims 8 and 9 to the conjugation buffer in the second flask, wherein said antibody differs from the antibody used in step d);
(f) preparing and adding aqueous solution comprising antibodies or antigens, wherein said antibodies are different from the antibody used in step d) and e) to the first flask;
(g) preparing and adding aqueous solution comprising antibodies or antigens complementary to the antibodies or antigens used in step (f) to the conjugation buffer in the second flask;
(h) adding colloidal gold solution into each flask;
(i) adding stabilizing buffer to each flask;
(j) concentrating each conjugate;
(k) adding a surfactant to the first conjugate and soaking glass fibre sheet conjugate pad into the conjugate;
(l) soaking another glass fibre sheet conjugate pad into the second conjugate;
(m) printing capturing/sample and control lines onto the membrane,
(n) laminating cards using the glass fibre sheet conjugate pad soaked with the first gold conjugate; and
(o) cutting cards into strips.

15. Use of a device according to any of claims 1 to 12 for the detection of a disease in at least one sample.

16. The use according to claim 15, wherein said sample was obtained from a human.

17. The use according to claim 16, wherein said sample is selected from the groups comprising of whole blood, serum, plasma, saliva, and urine

18. The use according to any of claims 15 to 17, wherein said disease detected in said sample is selected from the group consisting of HIV, Hepatitis A, Hepatitis B, Hepatitis C, H. pylori, Leishnnania, Schistosomiasis, Malaria, Pneumonia, Toxoplasmosis, Tuberculosis and Chlamydia infection.

19. Kit for detection of a disease comprising the device according to any of claims 1 to 11 and a manual.

20. The kit according to claim 19 further comprising an assay buffer.

21. The kit according to claim 20, wherein said assay buffer comprises a preservative.

## Patentansprüche

1. Immunochromatographische Schnelltestvorrichtung zur Detektion eines Targets in einer Probe, umfassend
(a) ein erstes Goldkonjugatfreisetzungkissen, umfassend ein erstes kolloidales Gold, konjugiert mit einem ersten Antikörper oder Antigen und wenigstens einem Oligonukleotid, und
(b) ein zweites Goldkonjugatfreisetzungskissen, umfassend ein zweites kolloidales Gold, konjugiert mit einem zweiten Antikörper oder Antigen und wenigstens einem Oligonukleotid, das komplementär zu dem wenigstens einen Oligonukleotid des ersten kolloidalen Goldkonjugats ist;
(c) ein Probenkissen;
(d) ein erstes Konjugatkissen, umfassend das erste Goldkonjugatfreisetzungskissen;
(e) ein zweites Konjugatkissen, umfassend das zweite Goldkonjugatfreisetzungskissen;
(f) eine Membran, umfassend eine Einfangtestzone und eine Negativkontrollzone;
und
(g) ein Absorptionskissen;
wobei die Einfangtestzone den zweiten Antikörper oder das zweite Antigen umfasst; wobei der zweite Antikörper, der in der Einfangtestzone immobilisiert ist, das Target von einer Stelle einfängt, die sich von derjenigen Stelle unterscheidet, die durch den ersten Antikörper eingefangen wird;
wobei beide Freisetzungskissen an unterschiedlichen Positionen in der Testvorrichtung angeordnet sind;
so dass das Target in der Probe durch das erste kolloidale Gold, das mit dem ersten Antikörper oder Antigen konjugiert ist, eingefangen werden wird, um einen Komplex "Target-erstes kolloidales Goldkonjugat" zu bilden, der Komplex dann durch den zweiten Antikörper oder das zweite Antigen, der/das in der Einfangtestzone immobilisiert ist, eingefangen werden wird und dann das zweite kolloidale Gold, das mit dem zweiten Antikörper oder Antigen konjugiert ist, freigesetzt werden wird und das Target von einer Stelle einfangen wird, die sich von derjenigen Stelle unterscheidet, die durch den ersten Antikörper eingefangen wird, und gleichzeitig mit dem (den) Oligonukleotid(en), das/die komplementär ist/sind mit dem (den) Oligonukleotid(en), das/die mit dem ersten kolloidalen Gold konjugiert ist/sind.

2. Immunochromatographische Schnelltestvorrichtung zur Detektion eines Targets in einer Probe, umfassend
(a) ein erstes Goldkonjugatfreisetzungskissen, umfassend ein erstes kolloidales Gold, konjugiert mit einem ersten Antikörper oder Antigen und wenigstens einem weiteren Antikörper oder Antigen, wobei der weitere Antikörper oder das weitere Antigen sich vom ersten Antikörper oder Antigen unterscheidet, und
(b) ein zweites Goldkonjugatfreisetzungskissen, umfassend ein zweites kolloidales Gold, konjugiert mit einem zweiten Antikörper oder Antigen und wenigstens einem weiteren Antikörper oder Antigen, wobei der weitere Antikörper oder das weitere Antigen spezifisch für den wenigstens einen weiteren Antikörper oder das wenigstens eine weitere Antigen des ersten kolloidalen Goldkonjugats sind;
(c) ein Probenkissen;
(d) ein erstes Konjugatkissen, umfassend das erste Goldkonjugatfreisetzungskissen;
(e) ein zweites Konjugatkissen, umfassend das zweite Goldkonjugatfreisetzungskissen;
(f) eine Membran, umfassend eine Einfangtestzone und eine Negativkontrollzone; und
(g) ein Absorptionskissen;
wobei die Einfangtestzone den zweiten Antikörper oder das zweite Antigen umfasst;
wobei der zweite Antikörper, der in der Einfangtestzone immobilisiert ist, das Target von einer Stelle einfängt, die sich von derjenigen Stelle unterscheidet, die durch den ersten Antikörper eingefangen wird;
wobei beide Freisetzungskissen an unterschiedlichen Positionen in der Testvorrichtung angeordnet sind;
so dass das Target in der Probe durch das erste kolloidale Gold, das mit dem ersten Antikörper oder Antigen konjugiert ist, eingefangen werden wird, um einen Komplex "Target-erstes kolloidales Goldkonjugat" zu bilden, der Komplex dann durch den zweiten Antikörper oder das zweite Antigen, der/das in der Einfangtestzone immobilisiert ist, eingefangen werden wird und dann das zweite kolloidale Gold, das mit dem zweiten Antikörper oder Antigen konjugiert ist, freigesetzt werden wird und das Target von einer Stelle einfangen wird, die sich von derjenigen Stelle unterscheidet, die durch den ersten Antikörper eingefangen wird, und gleichzeitig mit dem weiteren Antikörper oder Antigen, der/das spezifisch ist für den weiteren Antikörper und das weitere Antigen, der/das mit dem ersten kolloidalen Gold konjugiert ist.

3. Vorrichtung nach Anspruch 1 oder 2, wobei die Membran mittels eines Klebers an einer Tragerückschicht befestigt ist.

4. Vorrichtung nach einem der Ansprüche 1 bis 3, wobei die ersten und zweiten Goldkonjugatfreisetzungskissen zwischen dem Probenkissen und der Membran laminiert sind, wobei die zwei Goldkonjugate durch einen Teiler getrennt sind.

5. Vorrichtung nach einem der Ansprüche 1 bis 3, wobei das erste Goldkonjugatfreisetzungskissen zwischen dem Probenkissen und der Membran angebracht ist, während das zweite Goldkonjugatfreisetzungskissen sich im oberen Teil des Kunststoffgehäuses befindet.

6. Vorrichtung nach einem der Ansprüche 3 bis 5, wobei die Tragerückschicht eine Kunststoffrückschicht ist.

7. Vorrichtung nach einem der Ansprüche 1 bis 6, wobei die Membran Nitrocellulose-Membran ist.

8. Vorrichtung nach einem der Ansprüche 1 bis 7, wobei der erste oder zweite Antikörper ausgewählt ist aus der Gruppe, umfassend Maus-anti-HIV p24, Maus-anti-HBsAg, anti-hlgG, anti-Lipoarabinomannan, anti-H. pylori-Antigen, anti-Leishmania-Antigen, anti-Pneumonia-Antigen, anti-Malaria-Antigen, anti-Chlamydia-Antigen, Anti-Toxoplasma-Antigen, anti-Schistosoma-Antigen, HIV-1-Antikörper und HIV-2-Antikörper.

9. Vorrichtung nach einem der Ansprüche 1 bis 7, wobei das erste Antigen ausgewählt ist aus der Gruppe, umfassend Konjugat von HIV-Antigen, Konjugat von Hepatitis-C-Antigen, HIV-1-Antigen (HIV p160), HIV-2-Antigen (HIV p36), Hepatitis-B-Antigen, Lipoarabinomannan, H. pylori-Antigen, Toxoplasma-Antigen.

10. Vorrichtung nach einem der Ansprüche 1 bis 9, wobei die Kontrollzone einen nichtspezifischen Einfangantikörper und/oder ein nicht-spezifisches Antikörpereinfangprotein umfasst.

11. Vorrichtung nach Anspruch 10, wobei der nicht-spezifische Antikörper ausgewählt ist aus der Gruppe, bestehend aus anti-Maus-IgG, anti-Kaninchen-IgG, anti-Ziege-IgG, anti-Esel-IgG, anti-Schaf-IgG, anti-HIV p24, anti-Lipoarabinomannan, anti-H. pylori-Antigen, anti-Leishmania-Antigen, anti-Pneumonia-Antigen, anti-Malaria-Antigen, anti-Chlamydia-Antigen, anti-Toxoplasma-Antigen, anti-Schistosoma-Antigen, HIV-1-Antikörper und HIV-2-Antikörper.

12. Vorrichtung nach Anspruch 10, wobei das nicht-spezifische Einfangprotein ausgewählt ist aus entweder Protein A oder Protein G.

13. Verfahren zur Herstellung einer Vorrichtung nach einem der Ansprüche 1, 3 bis 12, umfassend die Schritte
(a) Herstellen einer kolloidalen Goldlösung;
(b) Herstellen eines Konjugationspuffers;
(c) Aufteilen eines Konjugationspuffers durch Verteilen desselben in einen ersten und einen zweiten Kolben;
(d) Zugeben eines Antikörpers nach Anspruch 8 zum Konjugationspuffer im ersten Kolben;
(e) Zugeben eines Antikörpers/Antigens nach den Ansprüchen 8 und 9 zum Konjugationspuffer im zweiten Kolben, wobei der Antikörper sich von dem in Schritt d) verwendeten Antikörper unterscheidet;
(f) Herstellen und Zugeben von mit Oligonukleotiden markierter wässriger BSA-Lösung zum ersten Kolben;
(g) Herstellen und Zugeben von mit komplementären Oligonukleotiden markierter wässriger BSA-Lösung zum Konjugationspuffer im zweiten Kolben;
(h) Zugeben kolloidaler Goldlösung in jeden Kolben;
(i) Zugeben von Stabilisierungspuffer zu jedem Kolben;
(j) Konzentrieren jeden Konj ugats;
(k) Zugeben eines Tensids zum ersten Konjugat und Tränken von Glasfaserschichtkonjugatkissen im Konjugat;
(l) Tränken eines weiteren Glasfaserschichtkonjugatkissens im zweiten Konjugat;
(m) Drucken von Einfang/Proben- und Kontrolllinien auf die Membran,
(n) Laminieren von Karten unter Verwendung des Glasfaserschichtkonjugatkissens, das mit dem ersten Goldkonjugat getränkt ist; und
(o) Zuschneiden der Karten zu Streifen.

14. Verfahren zur Herstellung einer Vorrichtung nach einem der Ansprüche 2 bis 12, umfassend die Schritte
(a) Herstellen einer kolloidalen Goldlösung;
(b) Herstellen eines Konjugationspuffers;
(c) Aufteilen des Konjugationspuffers durch Verteilen desselben in einen ersten und einen zweiten Kolben;
(d) Zugeben eines Antikörpers nach Anspruch 8 zum Konjugationspuffer im ersten Kolben;
(e) Zugeben eines Antikörpers/Antigens nach den Ansprüchen 8 und 9 zum Konjugationspuffer im zweiten Kolben, wobei sich der Antikörper von dem in Schritt d) verwendeten Antikörper unterscheidet;
(f) Herstellen und Zugeben von wässriger Lösung, die Antikörper oder Antigene umfasst, wobei die Antikörper von dem in Schritt d) und e) verwendeten Antikörper verschieden sind, zum ersten Kolben;
(g) Herstellen und Zugeben von wässriger Lösung, die Antikörper oder Antigene umfasst, die komplementär sind zu den in Schritt (f) verwendeten Antikörpern oder Antigenen, zum Konjugationspuffer im zweiten Kolben;
(h) Zugeben von kolloidaler Goldlösung in jeden Kolben;
(i) Zugeben von Stabilisierungspuffer zu jedem Kolben;
(j) Konzentrieren jeden Konjugats;
(k) Zugeben eines Tensids zum ersten Konjugat und Tränken von Glasfaserschichtkonjugatkissen im Konjugat;
(l) Tränken eines weiteren Glasfaserschichtkonjugatkissens im zweiten Konjugat;
(m)Drucken von Einfang/Proben- und Kontrolllinien auf die Membran;
(n) Laminieren von Karten unter Verwendung des Glasfaserschichtkonjugatkissens, das mit dem ersten Goldkonjugat getränkt ist; und
(o) Zuschneiden der Karten zu Streifen.

15. Verwendung einer Vorrichtung nach einem der Ansprüche 1 bis 12 zur Detektion einer Erkrankung in wenigstens einer Probe.

16. Verwendung nach Anspruch 15, wobei die Probe von einem Menschen erhalten wurde.

17. Verwendung nach Anspruch 16, wobei die Probe ausgewählt ist aus der Gruppe, die Vollblut, Serum, Plasma, Speichel und Harn umfasst.

18. Verwendung nach einem der Ansprüche 15 bis 17, wobei die Erkrankung, die in der Probe detektiert wird, ausgewählt ist aus der Gruppe, bestehend aus HIV, Hepatitis A, Hepatitis B, Hepatitis C, H. pylori, Leishmania, Schistosomiasis, Malaria, Pneumonie, Toxoplasmose, Tuberkulose und Chlamydia-Infektion.

19. Kit zur Detektion einer Erkrankung, der die Vorrichtung nach einem der Ansprüche 1 bis 12 und eine Anleitung umfasst.

20. Kit nach Anspruch 19, der weiter einen Testpuffer umfasst.

21. Kit nach Anspruch 20, wobei der Testpuffer einen Konservierungsstoff umfasst.

## Revendications

1. Dispositif d'essai rapide immunochromatographique pour la détection d'une cible dans un échantillon comprenant
(a) un tampon de libération d'un premier conjugué constitué d'or colloïdal, comprenant un premier conjugué constitué d'or colloïdal avec un premier anticorps ou antigène et au moins un oligonucléotide, et
(b) un tampon de libération d'un deuxième conjugué constitué d'or colloïdal, comprenant un deuxième conjugué constitué d'or colloïdal avec un deuxième anticorps ou antigène et au moins un oligonucléotide complémentaire à l'au moins un oligonucléotide du premier conjugué constitué d'or colloïdal ;
(c) un tampon à échantillon
(d) un premier tampon de conjugué comprenant ledit tampon de libération du premier conjugué constitué d'or colloïdal ;
(e) un deuxième tampon de conjugué comprenant ledit tampon de libération du deuxième conjugué constitué d'or colloïdal ;
(f) une membrane comprenant une zone d'essai de capture et une zone de contrôle négatif ; et
(g) un tampon absorbant ;
dans lequel ladite zone d'essai de capture comprend ledit deuxième anticorps ou antigène ;
dans lequel le deuxième anticorps immobilisé dans la zone d'essai de capture capture la cible à partir d'un site qui est différent du site capturé par le premier anticorps ;
dans lequel les deux tampons de libération sont situés à des positions différentes dans le dispositif d'essai ;
de sorte que la cible dans l'échantillon sera capturée par le premier or colloïdal conjugué au premier anticorps ou antigène pour former un complexe «cible-premier conjugué constitué d'or colloïdal », le complexe sera capturé ensuite par le deuxième anticorps ou antigène immobilisé dans la zone d'essai de capture, et ensuite le deuxième or colloïdal conjugué au deuxième anticorps ou antigène sera libéré et capture la cible à partir d'un site qui est différent du site capturé par le premier anticorps et en même temps avec le ou les oligonucléotides qui sont complémentaires au ou aux oligonucléotides conjugués au premier or colloïdal.

2. Dispositif d'essai rapide immunochromatographique pour la détection d'une cible dans un échantillon comprenant
(a) un tampon de libération d'un premier conjugué constitué d'or colloïdal comprenant un premier or colloïdal conjugué à un premier anticorps ou antigène et au moins un autre anticorps ou antigène, dans lequel ledit autre anticorps ou antigène est différent dudit premier anticorps ou antigène, et
(b) un tampon de libération d'un deuxième conjugué constitué d'or colloïdal, comprenant un deuxième or colloïdal conjugué à un deuxième anticorps ou antigène et au moins un autre anticorps ou antigène, dans lequel ledit autre anticorps ou antigène sont spécifiques à l'au moins un autre anticorps ou antigène du premier conjugué constitué d'or colloïdal ;
(c) un tampon à échantillon
(d) un premier tampon de conjugué comprenant ledit tampon de libération du premier conjugué constitué d'or colloïdal ;
(e) un deuxième tampon de conjugué comprenant ledit tampon de libération du deuxième conjugué constitué d'or colloïdal ;
(f) une membrane comprenant une zone d'essai de capture et une zone de contrôle négatif ; et
(g) un tampon absorbant ;
dans lequel ladite zone d'essai de capture comprend ledit deuxième anticorps ou antigène ;
dans lequel le deuxième anticorps immobilisé dans la zone d'essai de capture capture la cible à partir d'un site qui est différent du site capturé par le premier anticorps ;
dans lequel les deux tampons de libération sont situés à des positions différentes dans le dispositif d'essai ;
de sorte que la cible dans l'échantillon sera capturée par le premier or colloïdal conjugué au premier anticorps ou antigène pour former un complexe «cible-premier conjugué constitué d'or colloïdal», le complexe sera capturé ensuite par le deuxième anticorps ou antigène immobilisé dans la zone d'essai de capture, et ensuite le deuxième or colloïdal conjugué au deuxième anticorps ou antigène sera libéré et capture la cible à partir d'un site qui est différent du site capturé par le premier anticorps et en même temps avec l'autre anticorps ou antigène qui sont spécifiques à l'autre anticorps ou antigènes conjugué au premier or colloïdal.

3. Dispositif selon la revendication 1 ou 2, dans lequel ladite membrane est attachée au moyen d'un adhésif à un support.

4. Dispositif selon l'une quelconque des revendications 1 à 3, dans lequel lesdits tampons de libération du premier et du deuxième conjugués constitués d'or colloïdal sont stratifiés entre le tampon à échantillon et la membrane, dans lequel les deux conjugués constitué d'or sont séparés par un séparateur.

5. Dispositif selon l'une quelconque des revendications 1 à 3, dans lequel ledit tampon de libération du premier conjugué constitué d'or est attaché entre le tampon à échantillon et la membrane alors que le tampon de libération du deuxième conjugué constitué d'or se trouve dans la partie supérieure du coffret plastique.

6. Dispositif selon l'une quelconque des revendications 3 à 5, dans lequel ledit support est un support plastique.

7. Dispositif selon l'une quelconque des revendications 1 à 6, dans lequel ladite membrane est une membrane de nitrocellulose.

8. Dispositif selon l'une quelconque des revendications 1 à 7, dans lequel ledit premier ou deuxième anticorps est sélectionné dans le groupe composé d'un anticorps de souris anti-protéine p24 du VIH, d'un anticorps de souris anti-HBsAg, d'un anticorps anti-hlgG, d'un anticorps anti-Lipoarabinomannan, d'un anticorps dirigé contre l'antigène anti- H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

9. Dispositif selon l'une quelconque des revendications 1 à 7, dans lequel ledit premier antigène est sélectionné dans le groupe composé d'un conjugué d'un antigène du VIH, d'un conjugué d'un antigène de l'hépatite C, d'un antigène du VIH-1 (antigène p160 du VIH), d'un antigène du VIH-2 (antigène p36 du VIH), d'un antigène de l'Hépatite B, d'un antigène de Lipoarabinomannan, d'un antigène anti- H. pylori, d'un antigène de Toxoplasma.

10. Dispositif selon l'une quelconque des revendications 1 à 9, dans lequel ladite zone de contrôle comprend un anticorps de capture non spécifique et/ou une protéine de capture d'anticorps non spécifique.

11. Dispositif selon la revendication 10, dans lequel ledit anticorps non spécifique est sélectionné dans le groupe composé d'un anticorps anti-IgG de souris, d'un anticorps anti-IgG de lapin, d'un anticorps anti-IgG de chèvre, d'un anticorps anti-IgG de l'âne, d'un anticorps anti-IgG de mouton, d'un anticorps anti protéine p24 du VIH, d'un anticorps anti-Lipoarabinomannan, d'un anticorps dirigé contre l'antigène anti- H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

12. Dispositif selon la revendication 10, dans lequel ladite protéine de capture non spécifique sélectionnée est soit la Protéine A ou la Protéine G.

13. Procédé pour la fabrication d'un dispositif selon l'une quelconque des revendications 1, 3 à 12 comprenant les étapes qui consistent à
(a) préparer une solution constitué d'or colloïdal ;
(b) préparer un tampon de conjugaison ;
(c) répartir le tampon de conjugaison en le divisant dans un premier flacon et un deuxième flacon ;
(d) ajouter un anticorps selon la revendication 8 au tampon de conjugaison dans le premier flacon ;
(e) ajouter un anticorps/antigène selon les revendications 8 et 9 au tampon de conjugaison dans le deuxième flacon, dans lequel ledit anticorps est différent de l'anticorps utilisé dans l'étape d) ;
(f) préparer et ajouter une solution aqueuse de sérum-albumine bovin BSA à marquage d'oligonucléotides au premier flacon ;
(g) préparer et ajouter une solution aqueuse de sérum-albumine bovin BSA à marquage d'oligonucléotides complémentaire au tampon de conjugaison dans le deuxième flacon ;
(h) ajouter une solution constituée d'or colloïdal dans chaque flacon ;
(i) ajouter un tampon de stabilisation à chaque flacon ;
(j) concentrer chaque conjugué ;
(k) ajouter un tensioactif au premier conjugué et tremper un tampon conjugué en feuille en fibre de verre dans le conjugué ;
(l) tremper un autre tampon conjugué en feuille en fibre de verre dans le deuxième conjugué ;
(m) imprimer/capturer des lignes d'échantillon et de contrôle sur la membrane,
(n) stratifier des cartes en utilisant le tampon conjugué en feuille en fibre de verre trempé avec le premier conjugué constitué d'or ; et
(o) couper les cartes en bandes.

14. Procédé de fabrication d'un dispositif selon l'une quelconque des revendications 2 à 12, comprenant les étapes qui consistent à
(a) préparer une solution constituée d'or colloïdal ;
(b) préparer un tampon de conjugaison ;
(c) répartir le tampon de conjugaison en le divisant dans un premier flacon et un deuxième flacon ;
(d) ajouter un anticorps selon la revendication 8 au tampon de conjugaison dans le premier flacon ;
(e) ajouter un anticorps/antigène selon les revendications 8 et 9 au tampon de conjugaison dans le deuxième flacon, dans lequel ledit anticorps est différent de l'anticorps utilisé dans l'étape d) ;
(f) préparer et ajouter une solution aqueuse comprenant des anticorps ou antigènes, dans lequel lesdits anticorps sont différents de l'anticorps utilisé dans les étapes d) et e) au premier flacon ;
(g) préparer et ajouter une solution aqueuse comprenant des anticorps ou antigènes complémentaires aux anticorps ou antigènes utilisés dans l'étape (f) au tampon de conjugaison dans le deuxième flacon ;
(h) ajouter une solution constituée d'or colloïdal dans chaque flacon ;
(i) ajouter un tampon de stabilisation à chaque flacon ;
(j) concentrer chaque conjugué ;
(k) ajouter un tensioactif au premier conjugué et tremper un tampon conjugué en feuille en fibre de verre dans le conjugué ;
(l) tremper un autre tampon conjugué en feuille en fibre de verre dans le deuxième conjugué ;
(m) imprimer/capturer des lignes d'échantillon et de contrôle sur la membrane,
(n) stratifier des cartes en utilisant le tampon conjugué en feuille en fibre de verre trempé avec le premier conjugué constitué d'or ; et
(o) couper les cartes en bandes.

15. Utilisation d'un dispositif selon l'une quelconque des revendications 1 à 12 pour la détection d'une maladie dans au moins un échantillon.

16. Utilisation selon la revendication 15, dans laquelle ledit échantillon a été obtenu d'un être humain.

17. Utilisation selon la revendication 16, dans laquelle ledit échantillon est sélectionné dans le groupe composé de sang total, de sérum, de plasma, de salive et d'urine.

18. Utilisation selon l'une quelconque des revendications 15 à 17, dans laquelle ladite maladie détectée dans ledit échantillon est sélectionnée dans le groupe composé du VIH, de l'hépatite A, de l'hépatite B, de l'hépatite C, d'une infection H. pylori, de la Leishmania, de la Schistosomiasis, de la malaria, de la pneumonie, de la toxoplasmose, de la tuberculose et de l'infection de Chlamydia.

19. Trousse pour la détection d'une maladie comprenant le dispositif selon l'une quelconque des revendications 1 à 12 et un manuel.

20. Trousse selon la revendication 19 comprenant en outre un tampon d'essai.

21. Trousse selon la revendication 20, dans laquelle ledit tampon d'essai comprend un agent de conservation.
